# EUROPÄISCHE PATENTSCHRIFT

(11) **EP 0 385 062 B1**
(45) Veröffentlichungstag und Bekanntmachung des Hinweises auf die Patenterteilung: **27.07.1994**
(21) Anmeldenummer: 90100287.3
(22) Anmeldetag: 08.01.1990
(51) Int. Cl.: C07D 239/47

(54) **Verfahren zur Herstellung von Cytosinen**
Process for the preparation of cytosines
Procédé de préparation de cytosines

(30) Priorität: 03.03.1989 DE 3906855
(43) Veröffentlichungstag der Anmeldung: 05.09.1990
(73) Patentinhaber: HÜLS AKTIENGESELLSCHAFT, 45764 Marl (DE)
(72) Erfinder: Von Itter, Franz-Albert, Dr., D-5300 Bonn 1 (DE)

(56) Entgegenhaltungen:
- DE-A- 3 434 142
- CHEMICAL ABSTRACTS, Band 101, 1984, Seite 736, Zusammenfassung Nr. 191562w,Columbus, Ohio, US; & JP-A-59 93 059
- CHEMICAL ABSTRACTS, Band 101, 1984, Seite 736, Zusammenfassung Nr. 191563x,Columbus, Ohio, US; & JP-A-59 93 060

## Beschreibung

Die Erfindung betrifft ein Verfahren zur Herstellung von Cytosinen, die gegebenenfalls in der 5-Position substituiert sein können, aus β-Alkoxyacrylnitrilen und/ oder Dialkoxypropionitrilen und Harnstoff in Gegenwart von Alkoholaten und anschließender Neutralisation.

Es ist bekannt, die genannten Ausgangstoffe in Alkoholen als Lösungsmittel umzusetzen. Sehr bevorzugt wird in sekundären oder tertiären Alkoholen gearbeitet, wobei die Alkoholate und die Alkohole sich entsprechen. So beschreibt die EP-PS 0082 339 die Herstellung von Cytosin aus unsubstituierten Nitrilen in t-Butanol in Gegenwart von Kalium-t-butanolat. Die Ausbeute ist mit höchstens 62,3 % unbefriedigend. Diese Ausbeute ist nur mit den sekundären und tertiären Alkoholen erzielbar, die aber relativ teuer sind. Die niederen Alkohole Ethanol und Methanol zusammen mit deren Alkoholaten ergeben noch geringere Ausbeuten.

Nach DE-PS 3 434 142 wird in zwei Stufen gearbeitet. Zunächst entsteht ein Alkali- Harnstoffsalz in Suspension oder bevorzugt als fester Stoff. In dieser ersten Stufe sind gegebenenfalls auch niedere Alkohole verwendbar. Nachteilig ist die Notwendigkeit, in oder nach der ersten Stufe den Alkohol möglichst vollständig zu entfernen, da sonst die zweite Stufe unvollständig abläuft. Das erfordert höheren technischen Aufwand. In der zweiten Verfahrensstufe erfolgt die Umsetzung mit Nitrilen ebenfalls in Alkoholen, die bis 70 % Ausbeute nur dann ergibt, wenn die erste Stufe wie angegeben ausgeführt wird. Nachteilig ist die Zweistufigkeit des Verfahrens, der zweimalige Einsatz von Alkoholen und die Begrenzung der Temperatur auf 90 °C in der ersten Stufe, da im anderen Falle sich das Alkali-Harnstoffsalz zersetzt und die Ausbeute drastisch sinkt. Die Aufarbeitung des Lösungsmittels ist aufwendig, zumal nur wasserfreie Alkohole erneut verwendet werden können.

Es bestand daher die Aufgabe, den Verfahrensweg der Herstellung von Cytosinen zu verbessern und einfacher zu gestalten sowie gute oder bessere Ausbeuten zu erzielen. Nach Möglichkeit ist die Gewinnung des Harnstoffsalzes als gesonderte Stufe zu vermeiden. Ein weiteres Ziel ist, die Aufarbeitung der Lösungsmittel einfach auszuführen.

Überraschend wurde gefunden, daß die Umsetzung der genannten Ausgangsstoffe in unter den Reaktionsbedingungen inerten, mit Wasser wenig mischbaren, organischen Lösungsmitteln oder Lösungsmittelgemischen durchgeführt werden kann. Die Verwendbarkeit dieser weitgehend unpolaren aprotischen Lösungsmittel steht im direkten Widerspruch zu den bisher verwendeten Lösungsmitteln in Form der polaren Alkohole.

Gegenstand der Erfindung ist ein Verfahren zur Darstellung von Cytosinen der allgemeinen Formel I , die gegebenenfalls in 5-Stellung substituiert sein können, durch Reaktion eines β-Alkoxyacrylnitrils der Formel II und/oder eines β,β-Dialkoxypropionitrils der Formel III mit Harnstoff in Gegenwart eines Alkalialkoholates MeOR⁴ 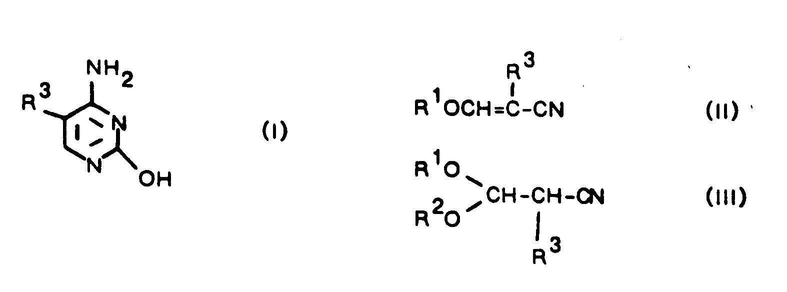
wobei R¹ und R² Alkylreste mit 1 bis 6 C-Atomen, die mit Alkoxy oder Halogen substituiert sein können, R³ Aralkylreste, H oder Alkylreste in der Bedeutung R¹ und R⁴ Alkylreste mit 1 bis 4 C-Atomen und Me ein Alkalimetall bedeuten
und Abscheidung sowie Gewinnung der entsprechenden Cytosine durch Neutralisation, bei Temperaturen von 0 bis 150 °C, dadurch gekennzeichnet, daß als Reaktionsmedium ein unter den Reaktionsbedingungen inertes, mit Wasser wenig mischbares, organisches Lösungsmittel oder Lösungsmittelgemisch verwendet wird, das bis zu 30% Alkohole als Nebenkomponente enthalten kann.

Überraschend können erfindungsgemäß Alkoholate beliebiger Alkohole, auch der niederen Alkohole Ethanol und ggf. Methanol verwendet werden.

Die Abtrennung des aus diesen Alkoholaten freigesetzten Alkohols in wasserfreier Form und getrennt von den verwendeten Lösungsmitteln ist einfach möglich. Die verwendeten Lösungsmittel sind in einfacher Weise zurückzugewinnen.

Als inerte, mit Wasser wenig mischbare, organische Lösungsmittel werden Kohlenwasserstoffe wie Hexan, Heptan oder die isomeren Oktane, Cyklohexan, Cycloheptan, Nitroethan, Chlorbenzol , Nitrobenzol und/oder Ether wie Anisol, Dibenzylether oder Diethylenglykolether, sehr bevorzugt Alkylaromaten wie Xylol, Cumol, Toluol oder Ethylbenzol, eingesetzt. Solchen Lösungsmitteln ist gemeinsam das aprotische Verhalten, d.h. eine geringe oder keine Dissoziation unter Freisetzung von Wasserstoffionen. Das erfindungsgemäße Verfahren erlaubt es, in einfacher Reaktionsführung Harnstoff und Alkoholat mit den Nitrilkomponenten II und III umzusetzen, ohne eine Zwischenisolierung eines Alkali-Harnstoffsalzes vorzunehmen. Während nach EP-PS 0082 339 bei Umsetzung der genannten Komponenten in niederen Alkoholen, wie Ethanol, drastische Ausbeuteverluste sich ergeben, kann bei Einsatz der oben beanspruchten Lösungsmittel auch in Gegenwart von niederen Alkoholen gearbeitet werden, sofern diese nur als Nebenkomponente vorhanden sind. Bei der Umsetzung von Harnstoff mit Alkoholat sollte das eingesetzte Lösungsmittel weniger als 30, sehr bevorzugt weniger als 20 Gew.% Alkohol enthalten. Ein weiterer Vorteil des vorliegenden Darstellungsverfahrens liegt darin, daß die Mischung aus Harnstoff und Alkoholat in den genannten Lösungsmitteln auch oberhalb der bei Alkoholen genannten kritischen Temperatur von 90 °C stabil ist.

Nach dem erfindungsgemäßen Verfahren wird die Reaktion im Temperaturbereich 0 bis 150 °C durchgeführt, wobei die Vermischung der Komponenten bei 0 bis 130 °C durchgeführt wird und zur Vervollständigung der Umsetzung im Temperaturbereich 60 bis 130 °C gearbeitet wird.

Bevorzugt erfolgt die Umsetzung in der Art, daß zunächst Harnstoff und Alkoholat bei Temperaturen von 0 bis 60 °C zusammengegeben werden und anschließend die β-Alkoxyacrylnitrile (II) und/oder die β,β-Dialkoxypropionitrile (III) bei Temperaturen von 60 bis 130 °C kontinuierlich oder portionsweise zugefügt werden.

Die Dosierung der Nitrilkomponente kann in 0,5 bis 2 Stunden erfolgen. Die Reaktionsdauer beträgt in Abhängigkeit von der Reaktionstemperatur und der Zugabezeit 0,5 bis 6 Stunden.

Die Alkoholate sind vorteilhaft Alkalialkoholate der niederen Alkohole, besonders bevorzugt des Natriums oder Kaliums. Der Alkoxyrest der Alkoholate besitzt vorzugsweise 1 bis 4, sehr bevorzugt 2 Kohlenstoffatome. Das Alkoholat kann bevorzugt suspendiert in der Reaktionslösung oder in dem Lösungsmittel eingesetzt werden. Das eingesetzte Molverhältnis von Harnstoff zu Alkoholat soll erfindungsgemäß 0,5 bis 5 zu 1, bevorzugt 1 bis 1,4 zu 1 betragen.

Das Molverhältnis von Harnstoff zur Nitrilkomponente II und/oder III soll erfindungsgemäß 0,5 bis 5 zu 1, bevorzugt 1 bis 2 zu 1 betragen. Unter Berücksichtigung der üblichen Stoffkosten ist es vorteilhaft, Harnstoff als billigste Komponente im Überschuß einzusetzen, um eine optimale Ausnutzung der anderen Reaktanden zu gewährleisten.

Bei der Umsetzung von Harnstoff mit Alkoholat sowie der Kondensation der Nitrilkomponenten zum Pyrimidin-System wird Alkohol gebildet. Zur Vervollständigung der Reaktion wird der im Reaktionsgemisch vorhandene Alkohol vorteilhaft während der Reaktion, spätestens vor der Aufarbeitung ganz oder teilweise entfernt. Durch diese wichtige und bevorzugte Maßnahme wird die Umsetzung vervollständigt und die Ausbeuten verbessert, sowie der Alkohol zurückgewonnen. Das bei der Reaktion gebildete Alkalisalz der Cytosine kann isoliert werden oder durch Zugabe von Säuren oder deren Anhydriden in die freien Cytosine überführt werden. Bei Verwendung von Schwefelsäure werden die entsprechenden Sulfate erhalten. Als weitere Methode, freie Cytosine darzustellen, kann die Verseifung eines zugesetzten Esters dienen.

Der Einsatz der beanspruchten Lösungsmittel, die mit Wasser wenig mischbar sind, ermöglicht zudem die Freisetzung und Isolierung der Cytosine nach einem vereinfachten Verfahren. Erfindungsgemäß wird zur Aufarbeitung der bei der Reaktion gebildete Feststoff, gegebenenfalls nach Zugabe von Wasser, abgetrennt.
Die Cytosine werden nach Phasentrennung mit Hilfe der obengenannten Neutralisationsmethoden aus der Wasserphase abgeschieden und isoliert. Nach Umkristallisation aus Wasser in Gegenwart von Aktivkohle können die Cytosine in hoher Reinheit in Ausbeuten von > 70 % erhalten werden.

Das abgetrennte organische Lösungsmittel trennt sich von Wasser und kann bei Einsatz der besonders bevorzugten Alkylaromaten ohne separate Aufarbeitung direkt für weitere Umsetzungen eingesetzt werden. Diese Rückführung erhöht die Produktausbeute auf über 77 %.

Auch die Mutterlaugen der Umkristallisation können zur wäßrigen Aufarbeitung nachfolgender Ansätze verwendet werden, wodurch sich die Ausbeute weiter steigern läßt.

Cytosine finden Verwendung zur Herstellung von Pharmazeutika, Agrochemikalien sowie als Fotochemikalie.

### Beispiel 1

78,1 g (1,3 m) Harnstoff und 81,7 g (1,2 m) Natriumethylat werden in 240 g Xylol suspendiert und auf Rückflußtemperatur erhitzt. Im Verlauf von 60 Minuten werden 143,2 g (1m) Cyanacetaldehyddiethylacetal zudosiert. Das Gemisch wird weitere 3 Stunden zum Sieden erhitzt und anschließend der bei der Reaktion gebildete Alkohol abdestilliert. Die verbleibende Suspension wird mit 500 g Wasser versetzt und die sich bildende wäßrige Produktphase von der Xylol-Phase abgetrennt. Durch Neutralisation der wäßrigen Phase mit 72 g (1,2 m) Essigsäure wird Cytosin ausgefällt. Eine anschließende Umkristallisation aus Wasser in Gegenwart von Aktivkohle ergibt 82,3 g (74,1 % d.Th.) reines Cytosin.

### Beispiel 2

In der abgetrennten Xylol-Phase aus Beispiel 1 werden 78,1 g (1,3 m) Harnstoff und 81,7 g (1,2 m) Natriumethylat suspendiert und mit 143,2 g (1m) Cyanacetaldehyddiethylacetal analog dem Beispiel 1 umgesetzt. Nach der oben genannten Aufarbeitung werden 85,8 g (77,2 % d.Th.) reines Cytosin erhalten.

### Beispiel 3

78,1 g (1,3 m) Harnstoff und 81,7 g (1,2 m) Natriumethylat werden in 240 g Xylol suspendiert und mit 23 g (0,5 m) Ethanol versetzt. Es wird auf Rückflußtemperatur erhitzt und im Verlauf von 1 Stunde werden 143,2 g (1 m) Cyanacetaldehyddiethylacetal zudosiert und weitere 3 Stunden zum Sieden erhitzt. Der in dem Reaktionsgemisch vorhandene Alkohol wird abdestilliert und der Rückstand mit 500 g Wasser versetzt. Aus der wäßrigen Phase wird Cytosin durch Zugabe von 72 g (1,2 m) Essigsäure ausgefällt, abfiltriert und aus Wasser in Gegenwart von Aktivkohle umkristallisiert. Es werden 81,6 g (73,5 % d.Th.) Cytosin erhalten.

### Beispiel 4

36 g (0,6 m) Harnstoff und 37,4 g (0,55 m) Natriumethylat werden in 150 g Toluol suspendiert und zum Sieden erhitzt. Während 30 Minuten werden 48,5 g (0,5 m) β-Ethoxyacrylnitril zudosiert und weitere 4 Stunden auf Rückflußtemperatur gehalten. Nach Abdestillation des Ethanols als Azeotrop mit Toluol wird mit 250 g Wasser versetzt und wie in Beispiel 1 aufgearbeitet. Nach Umkristallisation werden 39,6 g (71,4 % d.Th.) Cytosin isoliert.

### Beispiel 5

39 g (0,65 m) Harnstoff, 39,1 g (0,575 m) Natriumethylat und 71,6 g (0,5 m) Cyanacetaldehyddiethylacetal werden in 100 g Toluol zusammengegeben und 5 Stunden zum Sieden erhitzt. Anschließend wird das entstandene Ethanol als Azeotrop mit Toluol abdestilliert und der Rückstand in 250 g H₂O aufgenommen. Nach Abtrennung der wäßrigen Phase wird Cytosin durch Zugabe von 34,5 g (0,575 m) Essigsäure ausgefällt, filtriert und analog Beispiel 1 gereinigt. Als Ausbeute werden 34,1 g (61,3 d.Th. ) Cytosin erhalten.

### Beispiel 6

78,1 g (1,3 m) Harnstoff und 81,7 g Natriumethylat werden in 240 g Xylol suspendiert und analog Beispiel 1 mit 132,2 g (1m) Cyanacetaldehyddiethylacetal umgesetzt. Anschließend wird die Reaktionsmischung ohne Abdestillation des Alkohols mit 500 g Wasser versetzt und wie in Beispiel 1 aufgearbeitet. Es werden 74,6 g (67,1 % d .Th.) an reinen Cytosin erhalten.

### Beispiel 7

39 g (0,65 m) Harnstoff werden zusammen mit 37,4 g (0,55 m) Natriumethylat in 120 g Dibenzylether suspendiert und bei 95°C wird im Verlauf von 60 Minuten 48,5 g (0,5 m) β-Ethoxyacrylnitril zudosiert. Nach weiteren 3 Stunden Reaktion bei Rückflußtemperatur wird das entstandene Ethanol abdestilliert, der Rückstand mit 250 g Wasser versetzt und entsprechend Beispiel 1 aufgearbeitet. Es werden 39,2 g (70,6 % d .Th.) Cytosin erhalten.

### Beispiel 8

Beispiel 7 wird wiederholt, wobei als Lösungsmittel 120 g Chlorbenzol eingesetzt werden. Nach der oben genannten Aufarbeitung werden 35,7 g (64,3 % d.Th.) Cytosin erhalten.

### Beispiel 9

Zu 15,6 g (0,26 m) Harnstoff und 15,7 g (0,23 m) Natriumethylat in 50 g Xylol werden bei Rückflußtemperatur 25 g (0,2 m) 3-Ethoxy-2-ethylacrylnitril im Verlauf einer Stunde zudosiert und die Mischung wird weitere 3 Stunden zum Sieden erhitzt. Anschließend wird Ethanol abdestilliert und der Rückstand mit 120 g Wasser aufgenommen. Die wäßrige Phase wird abgetrennt und mit 13,8 g (0,23 m) Essigsäure versetzt. Das ausgefällte Produkt wird abgesaugt und aus Wasser in Gegenwart von Aktivkohle umkristallisiert. Es werden 18,4 g (66,3 % d.Th.) an reinem 5-Ethylcytosin erhalten.

## Patentansprüche

1. Verfahren zur Darstellung von Cytosinen der allgemeinen Formel I, die gegebenenfalls in 5-Stellung substituiert sein können, durch Reaktion eines β-Alkoxyacrylnitrils der Formel II und/oder eines β,β-Dialkoxypropionitrils der Formel III mit Harnstoff in Gegenwart eines Alkalialkoholates MeOR⁴ 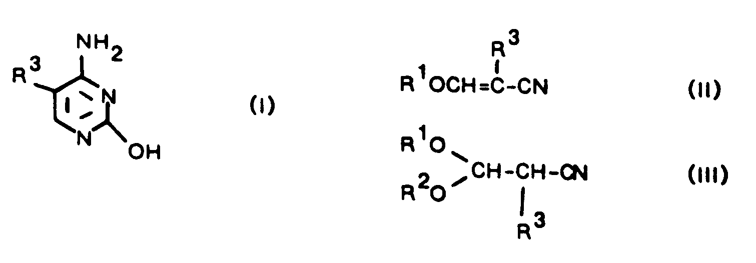 wobei R¹ und R² Alkylreste mit 1 bis 6 C-Atomen, die mit Alkoxy oder Halogen substituiert sein können, R³ Aralkylreste, H oder Alkylreste in der Bedeutung R¹ und R⁴ Alkylreste mit 1 bis 4 C-Atomen und Me ein Alkalimetall bedeuten
und Abscheidung sowie Gewinnung der entsprechenden Cytosine durch Neutralisation, bei Temperaturen von 0 bis 150 °C, dadurch gekennzeichnet, daß als Reaktionsmedium ein unter den Reaktionsbedingungen inertes, mit Wasser wenig mischbares, organisches Lösungsmittel oder Lösungsmittelgemisch verwendet wird, das bis zu 30% Alkohole als Nebenkomponente enthalten kann.

2. Verfahren nach Anspruch 1, dadurch gekennzeichnet, daß als Lösungsmittel bevorzugt Kohlenwasserstoffe und/oder Ether, sehr bevorzugt Alkylaromaten wie Xylol, Cumol, Toluol oder Ethylbenzol eingesetzt werden.

3. Verfahren nach einem der Ansprüche 1 oder 2, dadurch gekennzeichnet, daß die Reaktion in der Art bevorzugt durchgeführt wird, daß in einem ersten Reaktionsschritt Harnstoff und Alkoholat bei Temperaturen von 0 bis 60 °C zusammengegeben und anschließend β-Alkoxyacrylnitrile der Formel II und/oder β,β-Dialkoxypropionitrile der Formel III bei Temperaturen von 60 bis 130 °C kontinuierlich oder portionsweise zugefügt werden.

4. Verfahren nach mindestens einem der Ansprüche 1 bis 3, dadurch gekennzeichnet, daß Alkalialkoholate der niederen Alkohole, vorzugsweise des Natriums oder Kaliums, eingesetzt werden.

5. Verfahren nach mindestens einem der Ansprüche 1 bis 4, dadurch gekennzeichnet, daß das Molverhältnis von Harnstoff zu Alkoholat 0,5 bis 5 zu 1, vorzugsweise 1 bis 1,4 zu 1 beträgt.

6. Verfahren nach mindestens einem der Ansprüche 1 bis 5, dadurch gekennzeichnet, daß das Molverhältnis von Harnstoff zur Nitrilkomponente 0,5 bis 5 zu 1, vorzugsweise 1 bis 2 zu 1 beträgt.

7. Verfahren nach mindestens einem der Ansprüche 1 bis 6, dadurch gekennzeichnet, daß der bei der Reaktion gebildete Alkohol bereits während der Reaktion, spätestens vor der Aufarbeitung, ganz oder teil weise entfernt wird.

8. Verfahren nach mindestens einem der Ansprüche 1 bis 7, dadurch gekennzeichnet, daß der bei der Reaktion gebildete Feststoff, gegebenenfalls nach Auflösung oder teilweise Auflösung in Wasser, vom Lösungsmittel abgetrennt wird und dieses für weitere Umsetzungen wiederverwendet wird.

## Claims

1. Process for the preparation of cytosines of the general formula I which can be optionally substituted in the 5-position, by reaction of a β-alkoxyacrylonitrile of formula II and/or a β,β-dialkoxyproprionitrile of the formula III with urea in the presence of an alkali alcoholate MeOR⁴ 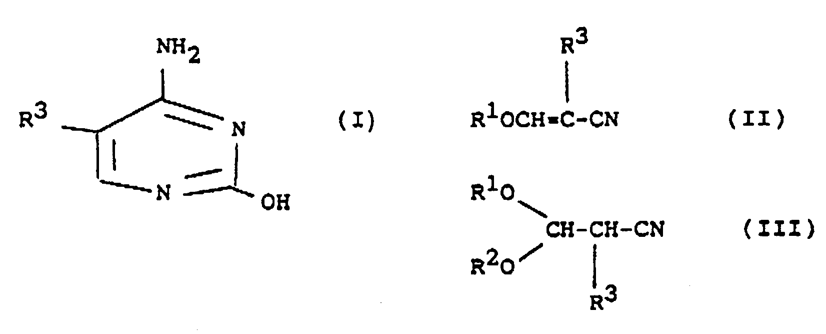 wherein R¹ and R² denote alkyl residues with 1 to 6 C-atoms which can be substituted with alkoxy or halogen, R³ denotes aralkyl residues, H or alkyl residues as indicated for R¹ and R⁴ denotes alkyl residues with 1 to 4 C-atoms and Me denotes an alkali metal, and separation off as wall as recovery of the corresponding cytosines by neutralisation, at temperatures of 0 to 150°C, characterised in that there is used as reaction medium an organic solvent or solvent mixture which is inert under the reaction conditions and is hardly miscible with water, which can contain up to 30% of alcohols as additional components.

2. Process according to claim 1, characterised in that preferably hydrocarbons and/or others, very preferably alkylaromatics like xylene, cumene, toluene or ethylbenzene, are employed as solvents.

3. Process according to one of claims 1 or 2, characterised in that the reaction is carried out preferably so that in a first reaction step urea and alcoholate are added together at temperatures of 0 to 60°C and then β-alkoxyacrylonitriles of the formula II and/or β,β-dialkoxypropionitriles of the formula III are supplied continuously or in portions at temperatures of 60 to 130°C.

4. Process according to at least one of claims 1 to 3, characterised in that alkali alcoholates of lower alcohols, preferably of sodium or potassium, are employed.

5. Process according to at least one of claims 1 to 4, characterised in that the molar ratio of urea to alcoholate amounts to 0.5 to 5:1, preferably 1 to 1.4:1.

6. Process according to at least one of claims 1 to 5, characterised in that the molar ratio of urea to the nitrile component amounts to 0.5 to 5:1, preferably 1 to 2:1.

7. Process according to at least one of claims 1 to 6, characterised in that the alcohol formed in the reaction is removed completely or partially already during the reaction, at latest before the working up.

8. Process according to at least one of claims 1 to 7, characterised in that the solid material formed in the reaction, if necessary after dissolution or partial dissolution in water is separated off from the solvent and this is reused for further reactions.

## Revendications

1. Procédé de préparation de cytosines de formule générale (I), qui peuvent éventuellement être substituées en position 5, par réaction d'un β-alcoxy acrylonitrile de formule II et/ou d'un β-β-dialcoxypropionitrile de formule III avec l'urée en présence d'un alcoolate alcalin MeOR⁴ 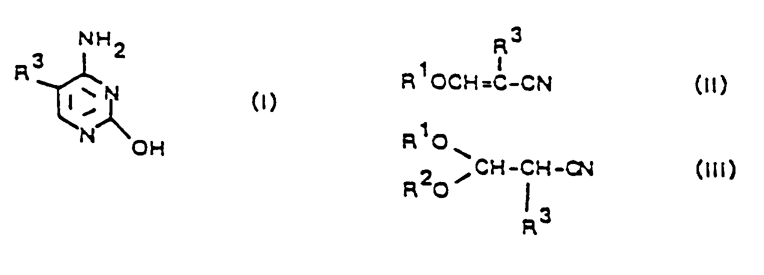 où R¹ et R² représentent des restes alkyles comportant 1 à 6 atomes de carbone, qui peuvent être substituée par un alcoxy ou un halogène; R³ représente des restes arylalkyles, H ou des restes alkyles ayant le sens de R¹, R⁴ représente des restes alkyles comportant 1 à 4 atomes de carbone et Me représente un métal alcalin, et séparation ainsi que récupération des cytosines correspondantes, par neutralisation à des températures de 0 à 150°C, procédé caractérisé en ce qu'on utilise comme milieu de réaction un solvant organique ou un mélange de solvants organiques inerte dans les conditions de réaction, peu miscible à l'eau, qui peut contenir jusqu'à 30 % d'alcools comme constituants secondaires .

2. Procédé selon la revendication 1, caractérisé en ce qu'on utilise, comme solvant, de préférence des hydrocarbures et/ou des éthers, de façon particulèrement préférée des hydrocarbures alkyl aromatiques comme le xylène, le cumène, le toluène ou l'éthylbenzène.

3. Procédé selon l'une des revendications 1 ou 2, caractérisé en ce qu'on effectue la réaction de préférence en regroupant, dans une première étape de réaction l'urée et l'alcoolate à des températures de 0 à 60°C, puis en introduisant en continu ou par portions les β-alcoxy acrylonitriles de formule II et/ou les β,β-dialcoxypropionitriles de formule III, à des températures de 60 à 130°C.

4. Procédé selon l'une au moins des revendications 1 à 3, caractérisé en ce qu'on utilise des alcoolates alcalins dérivant d'alcools inférieurs, avantageusement des alcoolates de sodium ou de potassium.

5. Procédé selon l'une au moins des revendications 1 à 4, caractérisé en ce que le rapport molaire entre l'urée et l'alcoolate vaut de 0,5 à 5 pour 1, avantageusement 1 à 1,4 pour 1.

6. Procédé selon l'une au moins des revendications 1 à 5, caractérisé en ce que le rapport molaire entre l'urée et le constituant nitrile vaut de 0,5 à 5 pour 1, avantageusement 1 à 2 pour 1.

7. Procédé selon l'une au moins des revendications 1 à 6, caractérisé en ce que l'alcool formé lors de la réaction est enlevé, totalement ou en partie, déjà pendant la réaction, au plus tard avant le traitement de purification.

8. Procédé selon l'une au moins des revendications 1 à 7, caractérisé en ce que le solide formé lors de la réaction, est enlevé du solvant, éventuellement après dissolution ou dissolution partielle dans l'eau, et ce solvant est utilisé à nouveau pour d'autres réactions.
